# EUROPEAN PATENT APPLICATION

(11) **EP 1 387 173 A1**
(43) Date of publication of application: **04.02.2004**
(21) Application number: 02017069.2
(22) Date of filing: 29.07.2002
(51) Int. Cl.: G01N 33/574, C07K 14/47

(54) **Method for improved diagnosis of cervical lesions based on detection of INK4a gene products**

(71) Applicant: MTM Laboratories AG, 69120 Heidelberg (DE)
(72) Inventor: Ridder, Rüdiger, 69198 Schriesheim (DE); Martin, Peter, 69251 Gaiberg (DE); Herkert, Matthias, 69120 Heidelberg (DE); Trunk-Gehmacher, Marcus, 97896 Freudenberg/Main (DE); Reichert, Anja, 69226 Nussloch (DE)

(57) **Abstract**

The present invention relates to a method for improved diagnosis of cervical lesions based on detection of gene products encoded by the INK4a gene locus. According to the present invention an improvement in diagnosis may be achieved by assessing the presence or absence or the level of overexpression of at least two different gene products encoded by the INK4a gene locus. Especially the discrimination of p16^{INK4a} overexpressing metaplasias from neoplastic p16^{INK4a} overexpressing lesions may be improved by determination of the level of other gene-products encoded by the INK4a locus such as p14ARF molecules in biological samples in the course of cytological testing procedures. The method thus enables for reduction of false positive results in the p16^{INK4a} based detection of anogenital lesions in cytological testing procedures.

## Description

The present invention relates to a method for improved diagnosis of cervical lesions based on detection of gene products encoded by the lNK4a gene locus. According to the present invention an improvement in diagnosis may be achieved by assessing the presence or absence or the level of overexpression of at least two different gene products encoded by the INK4a gene locus. Especially the discrimination of p16^{INK4a} overexpressing metaplasias from neoplastic or preneoplastic p16^{INK4a} overexpressing lesions may be improved by determination of the level of other gene-products encoded by the lNk4a locus such as p14ARF molecules in biological samples in the course of cytological testing procedures. The method thus enables for reduction of false positive results in the p15^{INK4a} based detection of anogenital lesions in cytological testing procedures.

The detection of the overexpression of p16^{INK4a} in biological samples has proven as a useful marker in the detection of anogenital lesions such as carcinoma of the uterine cervix (see WO00/01845; Klaes et al., Int. J. Cancer: 92, 276-284 (2001)). The method based on p16^{INK4a}-specific immunochemical staining allows for a sensitive and specific identification of dysplastic cells in tissue section and in cytological samples.

In immuno-histochemical examinations of tissues dysplastic and neoplastic cells can be stained using a p16^{INK4a} specific antibody mediated staining procedure. The histological diagnosis of neoplastic lesions can thus be supported by a staining procedure based on a molecular marker characteristic for transformation of cells in anogenital lesions. The diagnosis, whether or not cells are neoplastic, in these procedures is not solely based on the p16^{INK4a} specific staining, but does also rely on the histological information.

This is due to the fact, that in about 20-30% of samples metaplastic cells show some immunoreactivity with p16 ^{INK4a} specific antibodies, and thus are stained in the course of the procedures. Yet the staining pattern yielded from these metaplastic cells differs from the pattern, which renders from neoplastic lesions. Metaplastic cells give rise to a patchy or focal staining pattern, whereas neoplastic lesions give rise to diffuse staining pattern. Moreover the staining intensities of metaplastic cells are predominantly less than that of neoplastic cells.

The common methods used in screening tests for the early detection of dysplasias and/or neoplasias do not employ histology based tests, but do rather rely on cytological testing procedures. Yet especially in cases, when there is no histological information available concerning the architecture of tissues, such as for example in cytological examinations, testing for p16^{INK4a} overexpression alone may lead to false positive results. This is due to the fact, that those fractions of metaplastic cells expressing p16^{INK4a} at detectably elevated levels, may not be differentiated by means of a histologic criteria.

The percentage of cells showing overexpression of p16^{INK4a} increases in the course of emergence of dysplasias. So in neoplastic or preneoplastic stages, when only a restricted population of neoplastic or preneoplastic cells is present in samples the immunoreactivity of p16^{INK4a} may be weak. This weak immunoreactivity may be of about the level as the level caused by metaplastic cells. In later stages of dysplasias the overall immunoreactivity of p16^{INK4a} is stronger and so neoplastic lesions are easily discernible from metaplasias even in a cytological testing format. This might lead to cases, where the presence of metaplastic cells expressing p16^{INK4a} might be confused with the presence of neoplastic cells, and thus produces a false positive result.

Especially in screening tests, where the detection of early stages of neoplasias is desirable this condition is quite unpleasant. This is especially true, as the p16^{INK4a} based diagnosis has proven to be a valuable tool in histological examinations and the application in cytological based screening procedures would be able to enhance these established procedures.

To reduce false positive results in cytological testing formats and so to further enhance the fidelity of the p16^{INK4a} mediated diagnosis of anogenital lesions a method for discriminating the metaplasias from neoplastic and dysplastic lesions would be desirable. The problem in the art especially pertains to early stages of neoplasias, when the percentage of cells showing p16^{INK4a} overexpression is still at a level, that might be confused with levels of normally occurring p16^{INK4a} overexpressing proliferating metaplastic cells. Thus useful means for solving the present problem have to involve parameters, that characterize early stages of neoplasias of the anogenital tract. Any characteristics of dysplasias and/or neoplasias emerging during the progress of tumorigenesis, thus proving as diagnostic tools for high grade dysplasias, and are limited in early stages of tumorigenesis are not suitable for the method according to the present invention.

A method for the discrimination of metaplasias from neoplastic and preneoplastic lesions is provided within the embodiments claimed according to the present invention.

For supporting the discrimination of metaplasias from neoplastic lesions in testing procedures based on the overexpression of p16^{INK4a} a marker molecule would be desirable, that is expressed in neoplastic and/or preneoplastic cells and tissues and, which is not expressed in metaplastic cells.

A solution to the problem present in the art is provided by the methods claimed according to this invention. In the course of the experiments leading to the present invention the inventors have found, that a combination of detection of the presence or absence and/or the level of p16^{INK4a} in combination with at least one further expression product encoded by the INK4a gene locus, such as the p14ARF protein, may solve the problem present in the art.

The present invention relates to a method for discrimination of neoplastic, preneoplastic and/or dysplastic lesions from metaplasias comprising P16^{INK4a} overexpressing cells, in biological samples in a cytological testing procedure based on the detection of the presence or absence of cells expressing INK4a gene locus encoded gene-products in said biological samples. lNk4a gene locus encoded gene-products useful for the method disclosed herein are gene-products such as e.g. p14ARF. In one embodiment of the invention p14ARF protein or mRNA may serve as a marker for discrimination of metaplasias from early neoplastic or preneoplastic lesions in samples.

The INK4a gene locus as used in the context of the present invention is located on chromosome 9p21. The locus is alternatively nominated e.g. the lNK4a/ARF locus, CDKN2A gene, the MTS1 locus. The gene locus dealt with herein is that gene locus, that in one aspect encodes the gene product p16^{INK4a} protein, but is also known to encode for other gene product in alternative reading frames.

An gene product useful according to the present invention may be any molecule transcribed from a gene or any molecule translated from such a transcript. Thus gene product as used in the context of the present invention may comprise polynucleotides such as e.g. DNA or RNA and polypeptides such as proteins, proteoglycans, peptides etc. Expression product as used in the context of the present invention shall comprise any transcript of a gene locus in forward or reverse direction including any reading frames, splicing variants. Expression products as used herein shall thus comprise any alternative products encoded by the nucleic acids of a particular gene locus.

In one embodiment of the invention the cell cycle regulatory proteins are encoded by the INK4a gene. The cell cycle regulatory proteins as disclosed herein comprise any proteins encoded by alternative reading frames, arising from alternative splicing or usage of alternative promoter sequences of said gene. In one embodiment of the present invention the proteins exhibit molecular weights of about 5 to 40 kDa or any value in between and preferably of about 10 to 20 kDa or any value in between and most preferably of about 14 to about 19 kDa or any value in between respectively.

Discrimination as used in the context of the present invention shall comprise an assessment whether a sample is to be classified in one or another way. In a preferred embodiment of the invention the discrimination pertains to the assessment of a tissue or components thereof being neoplastic or being metaplastic. Thus the discrimination as used herein is a judgement about the growth properties of cells in a sample.

Neoplastic as used in the context of the present invention shall refer to dysplasias from mild to severe dysplasias and their precursory stages, as well as carcinoma such as carcinomas in situ or invasive carcinomas and disseminated tumor cells. Neoplastic as used herein shal comprise early and precursor stages of dysplasias and carcinomas. Metaplasia according to the method disclosed herein shall refer to a state during the differentiation of cells being intermediate between two differentiated states.

The discrimination according to the present invention is based on the presence or absence of cells expressing p14ARF and on the presence or absence of cells overexpressing P16^{INK4a} in said sample. The cells expressing p14ARF need not to be the same cells as those overexpressing P16^{INK4a} although the expression of both marker molecules may occur in the same cells.

Thus the presence of cells expressing p14ARF gene-products in a sample simultaneously with the presence of cells overexpressing p16^{INK4a} (other cells or the same cells coexpressing both markers) according to the present invention serves to discriminate neoplastic or preneoplastic lesions from metaplasias.

INK4a encoded gene-products as used in the context of the present invention shall be any mRNA transcribed from the lNK4a gene locus or any polypeptide translated from such an mRNA. INK4a gene-products suitable for the method according to the present invention are gene-products such as e.g. p16^{INK4a} and p14ARF.

The method for detection of the level of the INK4a encoded gene-products according to the present invention is any method, which may (but need not) be e.g. suited to detect even very small amounts of specific biological molecules in biological samples. The detection reaction according to the present invention is a detection either on the level of nucleic acids or on the level of polypeptides.

The INK4a gene-products may be detected using reagents that specifically recognise these molecules. The detection reaction for the INK4a gene-products may comprise one or more reactions with detecting agents either recognizing the initial marker molecules or recognizing the prior molecules used to recognize other molecules.

The detection of the different gene products may be performed in one reaction vessel or containment or in different containments simultaneously or subsequently in time. Thus the different gene products may be detected simultaneously in one cell coexpressing both products. Otherwise cell coexpressing the gene products may be used for separated detection reaction (separated in space or in time) to detect each a single marker in the cells. In another embodiment there might be cells expressing one or the other marker. The detection of the marker molecules in the different cells may as well be performed simultaneously or separately in time and/or space.

The detection reaction further may comprise a reporter reaction indicating the presence or absence and/or the level of the lNk4a gene-products. The reporter reaction may be for example a reaction producing a coloured compound, a bioluminescence reaction, a fluorescence reaction, generally a radiation emitting reaction etc..

In a preferred embodiment, different marker molecules may be recognized by agents, that produce different reporter signals, so that the signals referring to marker molecules could be distinguished. In one preferred embodiment of the invention the detection of the expression of the two or more lNK4a gene-products is carried out simultaneously. In this case the reporter reaction may for example employ different fluorescent labels for the different molecules detected.

Applicable formats for the detection reaction according to the present invention may be, blotting techniques, such as Western-Blot, Southern-blot, Northern-blot. The blotting techniques are known to those of ordinary skill in the art and may be performed for example as electro-blots, semidry-blots, vacuum-blots or dot-blots. Amplification reaction may also be applicable for the detection of e.g. nucleic acid molecules.

In one preferred embodiment of the invention the detection of the level of lNk4a gene-products is carried out by detection of the respective mRNA or fragments thereof present in the sample. The means for detection of nucleic acid molecules are known to those skilled in the art. The procedure for the detection of nucleic acids can for example be carried out by a binding reaction of the molecule to be detected to complementary nucleic acid probes, proteins with binding specificity for the nucleic acids or any other entities specifically recognizing and binding to said nucleic acids.

Probes as used in the context of the present invention may be any agent binding specifically to a molcule. In the case of nucleic acids a probe may be a oligonucleotide hybridising to a particular sequence. In one embodiment the probe may be e.g. a primer.

This method can be performed as well in vitro as directly in situ for example in the course of a detecting staining reaction. Another way of detecting the INK4a mRNAs in a sample performed in the method according to the present invention is an amplification reaction of nucleic acids, which can be carried out in a quantitative manner such as for example the polymerase chain reaction. In a preferred embodiment of the present invention real time RT PCR may be used to quantify the level of lNk4a mRNAs in samples of tumors.

In another preferred embodiment of the invention the detection of the level of INK4a gene-products is carried out by determining the level of expression of a protein. The determination of the INK4a gene-product on the protein level can for example be carried out in a reaction comprising a binding agent specific for the detection of the particular INK4a polypeptide.

The binding agents can be used in many different detection techniques for example in western-blot, ELISA or immuno-precipitation. Generally polypeptide binding agent based detection can be carried out as well in vitro as directly in situ for example in the course of an immuno-cytochemical staining reaction. Any other method for determining the amount of particular polypeptides in biological samples can be used according to the present invention.

Binding agents as used in the context of the present invention for the detection of the level of INK4a polypeptides such as p16^{INK4a} or p14ARF polypeptides may comprise antibodies and antigen-binding fragments, bifunctional hybrid antibodies, peptidomimetics containing minimal antigen-binding epitopes etc.

An antibody or antigen-binding agent is said to react specifically, if it reacts at a detectable level with a protein disclosed herein, and does not significantly react with other proteins. The antibodies according to the present invention may be monoclonal or polyclonal antibodies. As used herein, the term antibody or monoclonal antibody is meant to include intact molecules as well as antibody fragments. Moreover, antibodies of the present invention include chimeric, single chain, and humanized antibodies.

According to the present invention binding agents may be used isolated or in combination. By means of combination it is possible to achieve a higher degree of sensitivity. The term antibody, preferably, relates to antibodies which consist essentially of pooled monoclonal antibodies with different epitopic specificities, as well as distinct monoclonal antibody preparations.

Monoclonal antibodies are made from antigen containing fragments of the polypeptide of the invention using any of a variety of techniques known to those of ordinary skill in the art; see, e.g., Harlow and Lane, Antibodies: A Laboratory Manual, Cold Spring Harbor Laboratory, 1988. In one such technique, an immunogen comprising the antigenic polypeptide or a synthetic part thereof is initially injected into any of a wide variety of mammals (e.g., mice, rats, rabbits, sheep and goats). In this step, the polypeptides of this invention may serve as the immunogen without modification. Alternatively, particularly for relatively short polypeptides, a superior immune response may be elicited if the polypeptide is joined to a carrier protein, such as bovine serum albumin or keyhole limpet hemocyanin. The immunogen is injected into the animal host, preferably according to a predetermined schedule incorporating one or more booster immunizations, and the animals are bled periodically. Polyclonal antibodies specific for the polypeptide may then be purified from such antisera by, for example, affinity chromatography using the polypeptide coupled to a suitable solid support.

The lNk4a gene-products may according to the present invention be detected simultaneously. In this context simultaneously according to the present invention shall mean either literally at the same instant or within the same testing procedure, whereby the single detection steps are temporarily consecutive.

A sample according to the method of the present invention may comprise any sample comprising cells of anogenital origin. Samples may comprise e.g. secretions, smears, body fluids, and cell-samples.

In one embodiment of the present invention samples comprise cells of the uterine cervix. In a preferred embodiment of the present invention the sample of cervical cells may be prepared according to a classical Pap smear. In a further preferred embodiment of the present invention the sample may be prepared as a monolayer or thin layer preparation of the cytological specimen.

Preparation of a sample may comprise e.g. obtaining a sample of a tissue, of a body fluid, of cells from a patient. According to the present invention preparation of the sample may also comprise several steps of further preparations of the sample, such as preparation of dissections, spreading or applying the cells to be examined onto microscopic slides, preparation of tissue arrays, isolation of polypeptides or nucleic acids, preparation of solid phase fixed peptides or nucleic acids or preparation of beads, membranes or slides to which the molecules to be determined are coupled covalently or non-covalently.

The neoplastic lesions to which the method according to the present invention may be applied comprise any anogenital lesion, which is characterized by the overexpression of INK4a gene products. In one preferred embodiment of the present invention the anogenital lesion is a lesion of the uterine cervix.

Another aspect of the present invention is a testing kit for performing the method according to the present invention. The kit may be for example a diagnostic kit or a research kit.

A kit according to the present invention comprises at least an agent suitable for detecting the INK4a gene-products.

Thus a kit according to present invention may comprise:
a) reagents for the detection of the lNK4a gene-products
b) reagents and buffers commonly used for carrying out the detection reaction, such as buffers, detection-markers, carrier substances and others
c) lNK4a gene product samples for carrying out a positive control reaction

The reagents for the detection of the INK4a gene-products may include any agent capable of binding to the INK4a gene-products. Such reagents may include proteins, polypeptides, nucleic acids, peptide nucleic acids, glycoproteins, proteoglycans, polysaccharids or lipids.

The lNk4a gene-product samples for carrying out a positive control may comprise for example nucleic acids in applicable form, such as solution or salt, peptides in applicable form, tissue section samples or positive cells.

In a preferred embodiment of the invention the detection of the INK4a gene-products is carried out on the level of polypeptides. In this embodiment the binding agents may be for example antibodies specific for the INK4a gene-products or fragments thereof.

In another embodiment of the test kit the detection of the INK4a gene-products is carried out on the nucleic acid level. In this embodiment of the invention the reagent for the detection may be for example a nucleic acid probe or a primer reverse-complementary to said lNK4a nucleic acids.

The present invention provides a method for the discrimination of neoplastic and/or dysplastic and preneoplastic anogenital lesions, identifiable by assessment of the overexpression of p16^{INK4a}, from metaplastic cells, which may also detectably express p16^{INK4a}, in the course of cytological testing procedures. The method is based on the detection of expressed gene-products of two or more INK4a gene products.

Thus the problem to be solved was to provide a method for discrimination between neoplastic and metaplastic cells especially in early stages of neoplasias, when cytological diagnostic methods based on the p16^{INK4a} overexpression needs a further information for the identification of metaplastic cells.

Furthermore the present invention provides a kit for performing the method according to the present invention.

### Brief description of the drawings

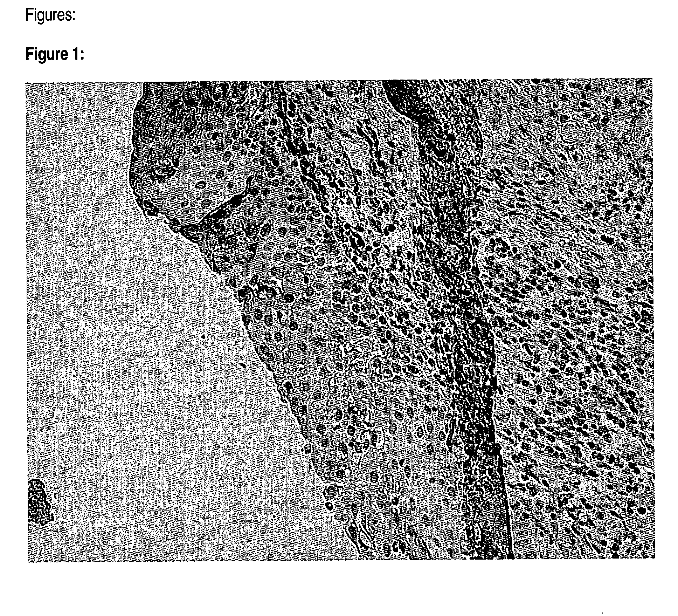

- Figure 1:: Cervix uteri, metaplastic area, the specimen has been subjected to an immunochemical staining reaction directed against p16^{INK4a}; the figure shows focal positive staining for p16^{INK4a}; for experimental details see example 1

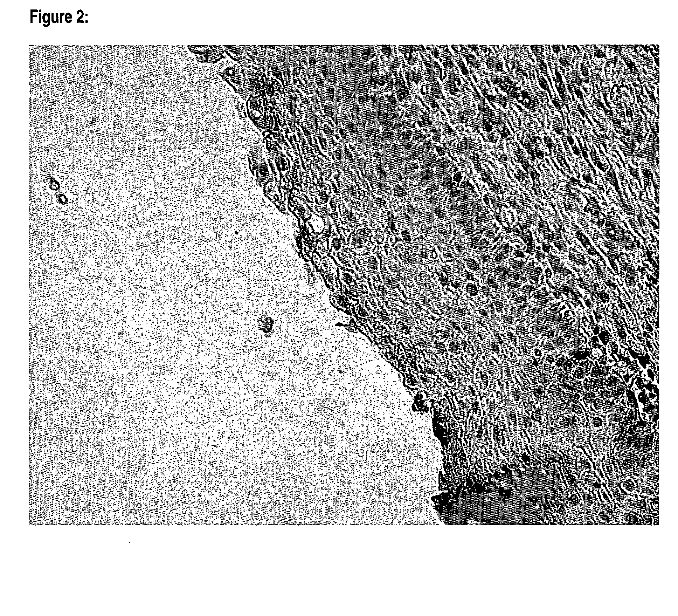

- Figure 2:: Cervix uteri, metaplastic area; the specimen has been subjected to an immunochemical staining reaction directed against p14ARF; the figure shows negative staining for p14ARF; for experimental details see example 1

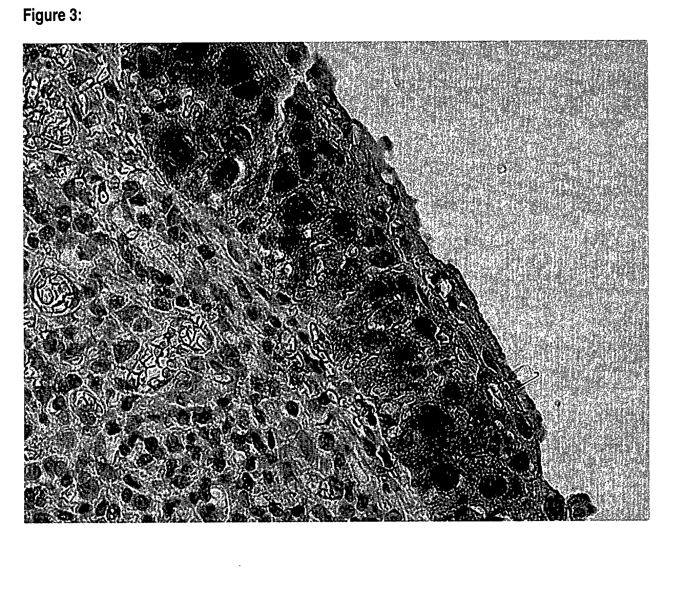

- Figure 3:: Cervix uteri, dysplastic area (CIN III); the specimen has been subjected to a immunochemical staining reaction directed against p16^{INK4a}; the figure shows diffuse positive staining for p16^{INK4a}; for experimental details see example 1

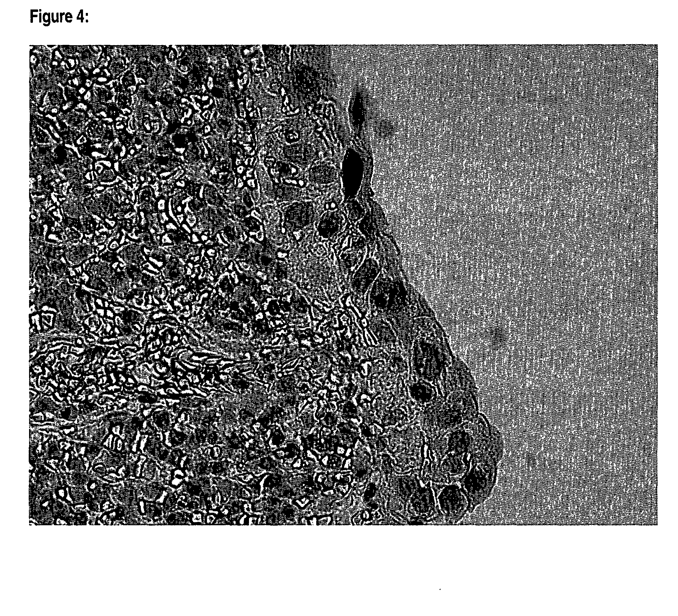

- Figure 4:: Cervix uteri, dysplastic area (CIN III); the specimen has been subjected to a immunochemical staining reaction directed against p14ARF; the figure shows positive nuclear staining for p14ARF; for experimental details see example1

The following examples are given for the purpose of illustration only and are not intended to limit the scope of the invention disclosed herein. For the purpose of illustration the methods disclosed herein are exemplified using histological preparations. The histological examples aid to judge whether the cells stained in the one or the other way are to be classified as neoplastic or metaplastic. The methods may easily be transferred to cytological specimens by altering the protocol in appropriate manner. These alterations are known to those of ordinary skill in the art.

### Example 1: Immunochemical detection of the overexpression of p16^{INK4a} and p14ARF in samples of the uterine cervix

Sections of formalin fixed, paraffin embedded tissue samples of the cervix uteri were immunocytochemically stained using antibodies specific for p16^{INK4a} and p14ARF.

The sections were rehydrated through incubation in xylene and graded ethanol, and transferred to Aqua bidest. Antigen Retrieval was carried out with 10mM citrate buffer ( pH 6.0 ) for p16^{INK4a}. Therefore the slides were heated in a waterbath for 40 min at 95 °C. For p14ARF the slides were heated in 1mmol EDTA (pH8.0) at 98°C for 20min. The slides were cooled down to RT for 20 minutes, transferred to washing buffer ( PBS / 0.1% Tween20 ) and finally the tissue sections were surrounded with a lipid-pencil.

For inactivation of endogenous peroxidase the samples are incubated with 3% H₂O₂ for 20 min at RT and afterwards washed in PBS / 0.1% Tween20 for 5 to 10 min.

The slides were then incubated with the primary antibody, mouse anti-human p16^{INK4a} antibody (1:25) for 30 min at RT, the slides were then rinsed with washing buffer and placed in a fresh buffer bath for 10 min. Excess buffer was tapped off and the specimen was covered with 100µl of visualization reagent for 30 min at RT. Slides were washed as before, and covered with 200 µl substrate-chromogen solution (DAB) for 10 min. Then slides were washed as before and counterstained for 3 min in a bath of hematoxylin. Residual hematoxylin was rinsed with destilled water, and specimens were mounted and coverslipped with an aqueous mounting medium.

Staining with the mouse anti-human p14ARF antibody was done as described before, except the antibody dilution was 1:100 and the incubation time was 1 h at RT.

The microscopic examination of the slides reveals, that cells immunoreactive with p16^{INK4a} together with cells immunoreactive with p14ARF protein can only be found in samples, that may microscopically be identified as samples of neoplastic lesions. Cells stained by the p16^{INK4a} specific reaction, that are originating from metaplasias, are not stained by the reaction specific for the p14ARF protein. The microscopic inspection of the p14ARF stained slides shows, that metaplastic cells are not immunoreactive with the antibodies directed against p14ARF protein. Samples containing dysplastic tissue areas in contrast comprise cells, that are immunoreactive with p14ARF and those immunoreactive with antibaodies directed against p16^{INK4a}. So in contrast to dysplasias in metaplasias no cells may be stained using the p14ARF specific antibody.

The results show, that the staining with reagents specific for p14ARF allows to discriminate p16^{INK4a} overexpressing metaplasias from dysplasias.

### Example 2: Detection of cells expressing p14ARF or p16^{INK4a} in samples of the uterine cervix by in situ hybridization

Smears of the uterine cervix can be semi-quantitatively analysed for the mRNA level of p16^{INK4a} and p14ARF in an in-situ staining reaction. The staining reaction is performed as follows:

For rehydration the spray-fixed smears are incubated in fresh 50% EtOH on a rocking device. The PEG film produced by the fixation procedure is removed by intensive rinsing. Following the smears are rinsed in aqua bidest. The smears are incubated with porteinase K (10pg/ml in PBS) for 10 min at 37°C. Then the slides are transferred to washing buffer ( PBS / 0.1% Tween20 ) and finally the area containing the cells was surrounded with a lipid-pencil.

The hybridization mixture is prepared by mixing 50 µl of ready to use hybridization buffer (DAKO A/S, Glostrup, Danmark) with about 5 - 10 pmol of the probes. The probes are fluorescein-labelled oligonucleotides of sequences complememtary to the respective mRNAs.

The hybridization mixture is heated to 95°C and afterwards equilibrated to 37°C. After the boiling procedure the smears are incubated with each 50 µl of the hybridization mixture for 4 hours at 42°C. The samples are washed in excess volumes of the wash buffers two times in 2 x SSC at 37°C for 15 min and once in 1 x SSC at 37 °C for 15 min Then the smears are rinsed two times at room temperature in 2 x SSC. Following this washing procedure the dissections are incubated for 30 min with blocking buffer (NEN, Blockingbuffer) at room temperature. Then follows 1 hour incubation with a 1:100 diluted (in Blocking buffer, see above) Anti-Fluorescein-alkaline phosphatase (DAKO A/S). The smears are then washed 2 times in 1 x PBS/0,1% Triton X-100 for 10 min at room temperature, followed by one wash step with 1 x PBS, 50 mM MgCl₂ (pH 9,2) for 10 min at room temperature. Then the staining reaction is performed with NBT/BCIP (Sigma) for 30 min to 2 hours at room temperature. The staining reaction is stopped by a short incubation with 1 mM EDTA in PBS. Finally the smears are dipped in H₂O_{dest.} and embedded with AquaTex (Merck). Then the stained dissections can be analysed microscopically.

Microscopic analysis reveals, that metaplasias comprise cells expressing p16^{INK4a} but no cells expressing p14ARF mRNA a detectable levels. In dysplastic lesions of the cervix uteri cells expressing P16^{INK4a} can be found and furthermore cells expressing p14ARF mRNAs at detectable levels.

This result indicates, that the method according to the present invention may be used for the discrimination of metaplasias and neoplastic lesions.

## Claims

1. A method for discrimination of metaplasias from neoplastic lesions in biological samples in the course of cytological testing procedures comprising
a. determining the presence or absence of cells overexpressing at least one lNK4a gene-product in said biological sample;
b. determining the presence or absence of cells expressing at least one further lNK4a gene-product in said biological sample;
c. assessing Simultaneous presence of cells expressing two different lNk4a gene-products or the presence of cells overexpressing only one INK4a gene-product alone;
d. wherein the simultaneous presence of cells expressing at least two different lNk4a gene-products is indicative for neoplastic lesions.

2. A method according to claim 1, wherein at least one of the INK4a gene-products has a molecular weight between 13 and 19 kDa.

3. A method according to claim 1, wherein at least one of the lNk4a gene-products is P16^{INK4a}.

4. A method according to claim 1, wherein at least one of the lNK4a gene-products is p14ARF.

5. A method according to any one of the preceding claims, wherein the lNk4a gene-product is a polypeptide or an RNA molecule.

6. The method according to any one of the preceding claims, wherein the neoplastic lesions are lesions of the anogenital tract.

7. The method according to claim 6, wherein the lesion of the anogenital tract is a lesion of the uterine cervix.

8. A method according to any preceding claim, wherein the biological sample is a sample containing cells of anogenital origin.

9. A method according to claim 8, wherein the cells are cells originating from the uterine cervix.

10. A method according to claim 9, wherein the biological sample is a cytological or histological preparation of the cervix uteri.

11. A method according to any one of the preceding claims, wherein the detection of the lNk4a gene-products is performed using at least one probe specifically recognizing the molecules to be detected.

12. A method according to claim 11, wherein the probe is detectably labelled.

13. A method according to claim 12, wherein the label is selected from the group consisting of a radioisotope, a bioluminescent compound, a chemiluminescent compound, a fluorescent compound, a metal chelate, or an enzyme.

14. A method according to any one of the claims 11 to 13, wherein the probe is a protein and/or a nucleic acid.

15. A method according to claim 14, wherein at least one probe is an antibody directed against a lNK4a encoded gene-product.

16. The method according to claim 15, which comprises an immuno-cytochemical staining procedure.

17. The method according to claim 14, wherein at least one probe is a nucleic acid specifically hybridizing to an INK4a gene-product.

18. The method according to claim 17, which comprises an in situ hybridization reaction.

19. The method according to claim 17, which comprises a nucleic acid amplification reaction.

20. The method according to claim 19, wherein the nucleic acid amplification reaction is PCR or LCR.

21. A method according to any of the preceding claims, wherein detection reactions using nucleic acid probes and polypeptide probes are carried out simultaneously.

22. A kit for performing the method according to any one of the preceding claims, which is a diagnostic kit or a research kit, comprising at least one or more probes for the detection of the presence or absence and/or the level of the overexpression of two or more INK4a gene-products in biological samples.

23. A kit according to claim 22, wherein the lNK4a gene products are selected from a group comprising p16^{INK4a} and p14ARF.

24. The kit according to claims 22 or 23 furthermore comprising at least one of the following
a. a p16^{INK4a} sample for carrying out a positive control reaction
b. a p14ARF sample for carrying out a positive control reaction
c. reagents for detection of the presence or absence and/or the level of p16^{INK4a}
d. reagents for detection of the presence or absence and/or the level of p14ARF
e. one or more samples of INK4a gene-products for carrying out positive control reactions
f. and one or more reagents for the detection of the presence or absence and/or the level of other lNK4a gene products.
